# EUROPÄISCHE PATENTSCHRIFT

(11) **EP 2 273 997 B1**
(45) Veröffentlichungstag und Bekanntmachung des Hinweises auf die Patenterteilung: **13.01.2016**
(21) Anmeldenummer: 09726923.7
(22) Anmeldetag: 24.03.2009
(51) Int. Cl.: A61K 35/14, A61K 35/16, A61K 35/28, A61K 35/19, A61K 38/18, A61K 38/19, A61K 38/48, A61K 33/06, A61K 31/74, A61P 17/02, A61P 19/00, A61P 19/04

(54) **VERFAHREN UND ZUSAMMENSETZUNG ZUR REGENERATION VON GEWEBE MIT HILFE VON STAMM- ODER KNOCHENMARKZELLEN**
METHOD AND COMPOSITION FOR REGENERATING TISSUE WITH THE AID OF STEM OR BONE MARROW CELLS
PROCÉDÉ ET COMPOSITION DESTINÉS À RÉGÉNÉRER DES TISSUS À L'AIDE DE CELLULES SOUCHES OU DE CELLULES DE MOELLE OSSEUSE

(30) Priorität: 31.03.2008 EP 08006238
(43) Veröffentlichungstag der Anmeldung: 19.01.2011
(73) Patentinhaber: Bader, Augustinus, 04668 Parthenstein / OT Klinga (DE)
(72) Erfinder: Bader, Augustinus, 04668 Parthenstein / OT Klinga (DE)
(74) Vertreter: Benz, Jürgen
(86) Internationale Anmeldenummer: PCT/EP2009/002135
(87) Internationale Veröffentlichungsnummer: WO 2009/121503

(56) Entgegenhaltungen:
- WO-A-2004/001023
- WO-A-2005/063965
- WO-A-2005/070450
- US-A1- 2004 191 215
- US-B1- 6 541 024
- KINOSHITA KAZUHIKO ET AL: "Promoted new bone formation in maxillary distraction osteogenesis using a tissue-engineered osteogenic material" JOURNAL OF CRANIOFACIAL SURGERY, BURLINGTON, CA, Bd. 19, Nr. 1, 1. Januar 2008 (2008-01-01), Seiten 80-87, XP009123049 ISSN: 1049-2275
- YAMADA YOICHI ET AL: "A novel approach to periodontal tissue regeneration with mesenchymal stem cells and platelet-rich plasma using tissue engineering technology: A clinical case report" THE INTERNATIONAL JOURNAL OF PERIODONTICS & RESTORATIVE DENTISTRY, QUINTESSENCE PUBL, Bd. 26, Nr. 4, 1. August 2006 (2006-08-01), Seiten 363-369, XP001525060 ISSN: 0198-7569

## Beschreibung

Die Erfindung betrifft eine neuartige polymerisierbare Zusammensetzung umfassend Blut, Blutplättchen oder Blutplasma, sowie Stammzellen oder Zellen des Knochenmarks, sowie gegebenenfalls weitere Zellen des Blutes, des Fettgewebes oder eines Gewebes, welches aus dem aufzubauenden oder zu regenerierenden Zielgewebe stammt oder mit diesem übereinstimmt. Mit dieser Blut/Stammzellenpräparation kann in sehr kurzer Zeit zielgerichtet ein beliebiges Zielgewebe hergestellt werden.

Bei vielen Verletzungen und Erkrankungen bestehen Defekte im Körper, die dieser auf Grund einer evolutionären Barriere nicht reparieren kann. Das Ziel dieser Erfindung ist es, eine "Kopiervorlage" in ihrer Herstellungsfähigkeit für das "Engineering" von Gewebe machbar werden zu lassen und auch ein Verfahren aufzuzeigen, wodurch ein beliebiges Zielgewebe hergestellt werden kann.

Derartige Mischungen können unter dem Einfluss endogener oder exogener Polymerisierungsfaktoren, wie Thrombin, Calcium-Ionen oder Zelldetritus zu viskosen Gelen polymerisieren, welche sehr vorteilhaft für die Entwicklung und Differenzierung der Stamm- bzw. Knochenmarkzellen zu gewebespezifischen Zellen sind.

Derartige Polymerisate, die insbesondere zusätzlich Erythropoietin (EPO), Analoga oder Derivate von EPO (z.B. in carbamylierter Form) oder Peptidsequenzen von EPO und oder Thrombopoietin, oder Thrombin enthalten, zeigen hervorragende Eigenschaften bei ihrem Einsatz für Geweberegeneration oder bei der Regeneration von Knochendefekten. Alternativ kann auch GM-CSF oder G-CSF alleine oder in Kombination mit EPO verwendet werden.

Die Erfindung betrifft ferner die Polymerisate zur Verwendung in einem Verfahren zur Regeneration und Genese von Gewebe mit Hilfe dieser Polymerisate aus Blutkomponenten, Stammzellen / Knochenmark und vorzugsweise Zellwachstum und Zelldifferenzierung fördernden Faktoren und Substanzen, insbesondere EPO und seine biologisch aktiven Derivate und / oder Fragmenten, sowie GM-CSF oder G-CSF sowie gegebenenfalls Vitaminen, beispielsweise Vitamin C, oder Hormonen. Embryonale, neo-natale (aus der Nabelschnur) und adulte Stammzellen gelten als die Hoffnungsträger für neue Therapieformen, mit denen die Regeneration von zerstörten Geweben und Organen möglich werden soll. Diese Zellen besitzen offenbar tatsächlich das Potential zur Reparatur zerstörter Gewebeteile, doch die dem zugrunde liegenden Mechanismen sowie die praktische Anwendbarkeit in Bezug auf spezielle Gewebe werden nach wie vor kontrovers diskutiert.

Unter der Bezeichnung "Stammzellen" wird eine uneinheitliche Gruppe von Zellen zusammengefasst, die mindestens die folgenden zwei Eigenschaften gemeinsam haben: Stammzellen sind Vorläuferzellen von hoch differenzierten Zellen. Nach einer Teilung der Stammzellen können die Tochterzellen, so die Lehrmeinung, entweder wieder zu Stammzellen werden oder sich gewebespezifisch, z.B. zu Herz-, Nerven-, Haut- oder Muskelzellen, differenzieren.

Stammzellen treten zuerst in der frühen Embryonalentwicklung auf. Bereits die befruchtete Eizelle (Zygote) stellt eine omnipotente Stammzelle dar, die die frühen Embryonalstadien durchläuft und aus der sich später alle Gewebe des menschlichen Körpers bilden. So bilden sich unter dem Einfluss bestimmter Wachstumsfaktoren während der Embryogenese beispielsweise Stammzellen des embryonalen Bindegewebes (mesenchymale Zellen) zu Muskelzellen um. Je weiter die Spezialisierung der Tochterzellen einer Stammzelle voranschreitet, desto stärker wird das Spektrum ihrer Differenzierungsmöglichkeiten in verschiedene Gewebe eingeschränkt.

Andere Stammzellen, die so genannten adulten Stammzellen, spielen hingegen während des gesamten Lebens eine wichtige Rolle insbesondere bei der Geweberegeneration und - reparatur. Sie erhalten die Funktionsfähigkeit von Geweben und Organen aufrecht, indem sie differenzierte Zellen nachliefern und beschädigte oder abgestorbene Zellen ersetzen. Beispielsweise sorgen Stammzellen des Knochenmarks für den Nachschub kurzlebiger Blutzellen.

Bis vor kurzem war man der Auffassung, dass adulte Stammzellen nicht nur Zellen des entsprechenden Organs, in dem sie aufzufinden sind, hervorbringen können, sondern auch Zellen anderer Gewebe oder Organe. Aus dem Knochenmark wurden zum Beispiel nicht nur neue Blutzellen abgeleitet, sondern auch Zellen verschiedener Körpergewebe, wie Knochen, Knorpel, Sehnen, Muskeln, Leber.

Die Meinung, dass sich adulte Stammzellen aus dem Knochenmark in jede beliebige differenzierte Zelle umwandeln kann, ist aber basierend auf jüngeren Ergebnissen umstritten, die zeigen, dass bei mesenchymalen Stammzellen ein solches Potential zur Transdifferenzierung nur ansatzweise oder nur unter bestimmten Voraussetzungen gegeben ist: In vielen Fällen, in denen aus mesenchymalen Stammzellen tatsächlich voll funktionsfähige spezialisierte Gewebezellen, z. B. Skelettmuskelzellen hervorgegangen sind, erfolgte dies durch Fusion der Stammzellen mit vorhandenen, bereits fertigen differenzierten Zellen. Experimente zeigen zwar, dass diese Zellen z.B. eine Reihe Herz- und Skelettmuskulatur-spezifischer Gene exprimieren, wenn sie zusammen mit bestimmten Wachstumsfaktor-produzierenden Zellen kultiviert wurden. Doch obwohl bei den Zellen auch morphologische Änderungen zu beobachten waren, fanden sich letztlich keine voll funktionsfähigen Muskelzellen.

So gelang es bisher nicht, eine Stammzellentherapie beim Menschen durchzuführen, welche zu einer echten Regeneration von speziellem Gewebe in situ führt. Beispielsweise führt die Transplantation von adulten Stammzellen in das Herz zur verstärkten Angiogenese aber offensichtlich nicht zum Aufbau von Herzmuskelgewebe.

Daher verwendet man bei der Regeneration von Gewebe sehr oft Ausgangszellen, die bereits entsprechend spezialisiert bzw. differenziert sind. Beispielsweise werden für die Regeneration von Knorpelgeweben entsprechend dem Stand der Technik Matrices wie z. B. Fibrin-Heterologen wie auch autologen Ursprungs aber auch z. B. Rattenschwanzkollagen verwendet. In diese polymeren Matrices werden üblicherweise Knorpelzellen artikulären Ursprungs hineingegeben. Diese werden nach einer Biopsie aus dem betroffenen Kniegelenk eines Patienten gewonnen und *lege artis* unter Verwendung von ex vivo Expansionsverfahren auf diese Matrizes aufgesiedelt. Alternativ können in bekannter Weise die Knorpelzellen direkt in den Alginat-, Fibrin- oder Kollagenmatrices tierischen Ursprungs gezüchtet und nach einigen Wochen Kultivierungszeit transplantiert werden. Ähnliche Verfahren sind auch für die Herstellung andere Gewebe spezifischen Zellen bekannt.

Ein Nachteil dieser Verfahren besteht darin, dass die notwendigen längeren Vorkultivierungszeiten zu De-Differenzierungsprozessen der ausgewählten spezifischen Zellen führen, welche dann ungewünschte Eigenschaften annehmen. Beispielsweise begünstigen derartige fehlgeleitete Differenzierungsprozesse von Knorpelzellen die Entstehung des nicht erwünschten fibrotischen statt eines hyalinen Knorpels.

Ein weiterer wesentlicher Nachteil der bislang üblichen Methoden besteht darin, dass die heute verwendeten polymeren Matrices das Neu- und Umbilden des Gewebes nicht begünstigen und unerwünschte Zellaktivierung auslösen. Überdies ergeben sich auch nicht selten immunologische Unverträglichkeiten für den Empfänger.

Die genannten Nachteile können nun erfindungsgemäß durch Verwendung von Stammzellen anstelle von ausdifferenzierten Zellen vermieden werden, wobei Stammzellen, nicht wie bislang zumeist direkt in das zu regenerierende Zielgewebe inkorporiert werden, sondern mittels einer Matrix, welche im wesentlichen aus polymeren Blut oder Blutplasma oder polymerisierbaren Blutplättchenkonzentraten oder -präparaten besteht, in welche die Stammzellen oder deren Vorläuferzellen vor der Polymerisation eingebettet worden sind.

So kann überraschenderweise gezeigt werden, dass der Einsatz derartiger, insbesondere adulte Stammzellen haltige Polymerisate auf Basis von Blut, bzw. dessen zelluläre Bestandteile (rote und weiße Blutkörperchen, Blutplättchen) oder nicht-zelluläre Bestandteile (Proteine, Lipide, Zucker etc.) in Bezug auf die Ausbeute und Qualität der erhaltenen differenzierten Spezialzellen wesentlich höher ist als bei Anwendung der bekannten Verfahren wie oben beschrieben, inklusive der Methoden, welche Gewebezellen als Ausgangszellen benutzen.

Überraschenderweise ist dieser Effekt auch gegenüber entsprechenden Blutpräparationen zu beobachten, welche nicht zuvor polymerisiert wurden. Polymerisiertes Blut oder Blutplasma übt also im Gegensatz zu flüssigen Blutproben auf die Stammzellen einen sehr positiven Effekt in Bezug auf deren Fähigkeit aus, zu gewebespezifischen Zellen zu differenzieren und in differenzierter Form sich zu vermehren.

Insbesondere die Polymerisationsvermittler wie Thrombin, Ca++ oder synthetische oder biologische Matrixfragmente wie z.B. Peptide, welche Kollagensequenzen oder auch RDG Sequenzen enthalten, sind nicht nur für die Polymerisation sondern auch für den biologischen Effekt in der 3D-Struktur des Blutes, der Plasmastruktur, oder der Struktur der Blutplättchenpräparation nach der Polymerisation verantwortlich.

Der Grund liegt darin, dass hierdurch eine Reihe von Wachstumsfaktoren freigesetzt werden, die für die Stammzellen eine unmittelbare stimulierende Wirkung haben. Hierzu zählen EGF aber auch TGF beta. Neben diese Faktoren stellen aber Zellmembranbestandteile, Matrixbestandteile, Zellinhaltsbestandteile und komplexe mineralische Elemente (Na, K, Cl, Mg, Zink) Substanz-Kontiminationen dar, die in normalen synthetischen Materialien üblicherweise nicht vorkommen Zusätzlich können auch Fibrin-Polymerisate diese Leistungen hinsichtlich der Komplexität des Mikromilieus nicht erfüllen.

Ferner wurde erfindungsgemäß festgestellt, dass dieser Effekt polymerisierten Blutes insbesondere bei Anwesenheit von Faktoren auftritt, welche die Freisetzung, Vermehrung oder Differenzierung der Stammzellen fördern. Dies sind nicht nur die üblichen bei den Stammzellenherstellungsverfahren eingesetzten Hormone, Vitamine oder Wachstumsfaktoren, wie beispielsweise GM-CSF, G-CSF sondern überraschenderweise und im besonderen Maße auch Erythropoietin (EPO), deren Analoga, carbamylierten Formen oder auch Peptidfragmente die Sequenzen der natürlichen Substanzen enthalten.

Erfindungsgemäß wurde nämlich gefunden, dass in der 3-D Struktur des erfindungsgemäßen Blutgels in Verbindung mit den Stammzellen bzw. Progenitoren ein künstliches und potenziertes Wundmilieu geschaffen wird, welches in Verbindung mit unter diesen Bedingungen auftretendem Sauerstoffmangel und dem lokalen Auftreten von freigesetzten Akutphasenzytokinen, wie z.B. Interleukin-6 (IL-6), Interleukin.1 (IL-1) und Tumor Necrosis Faktor (TNF), eine Kaskadenreaktion in Bezug auf die Stammzellen auslöst. Die Co-Stimulierung mit EPO / Derivaten oder Analoga ergibt hierbei einen wesentlichen permissiven Effekt, der die eigentlichen Effektorzellen (Stammzellen und Progenitoren) in die Gewebedifferenzierung translatieren lässt.

Ein weiterer Vorteil der Gelstruktur und des hierdurch indizierbaren Wandels von einer flüssigen (sol) zu einer verfestigten (polymerisierten) Gel-Struktur liegt in der Modellierbarkeit in einer Wundhöhle oder einem Wunddefekt, sowie in der generellen topischen Applizierbarkeit durch die klebende Wirkung der Verbindungen. Hierdurch können Stammzellen hocheffektiv in die Nachbarschaft eines Wundgrundes eingebracht werden und somit einfach Defekte aufgefüllt werden.

Auch bei chronischen Erkrankungsformen, wie z.B. einer Querschnittverletzung im chronischen Zustand, bei der die örtliche Trauma-Situation schon abgeklungen ist, oder auch bei kosmetischen Eingriffen im Bereich der Haut zur Narbenkorrektur oder zum Aufbau von Füllungsdefekten, bei der Brustrekonstruktion mit wiederholten subkutanen Injektionen oder auch topisch im Abstand von 3-4 Tagen, kann durch diesen erfindungsgemäßen Applikationscocktail die Trauma-Situation lokal begrenzt appliziert werden. Hierdurch wird in einem Mikrobereich ein ideales Umfeld für die Stammzellstimulierung zur Einleitung eines ortspezifischen Gewebeneuaufbaus geschaffen. Durch die Permeabilität des 3-D Konstruktes können zusätzlich hormonelle und parakrine Signale aus der Umfeld auf die Stammzellen einwirken und diese anregen.

Im Unterschied zur natürlichen Situation einer Wunde, wird durch die Kombination dieser Faktoren eine Potenzierung der Effekte ausgelöst, die eine beschleunigte Wundheilung bis zu etwa 50% bewirken kann. Ein weiterer Vorteil der Gelstruktur ist neben der autologen Basis die vollständige Komplementierbarkeit mit synthetischen Komponenten (BMPs, PDGF, EPO, GCSF, GM-CSF oder synthetischen Matrixkomponenten.

Blutpolymerisate, Knochenmarkpolymerisate, oder Blutplättchenkonzentrate, welche Stammzellen und vorzugsweise zusätzlich EPO und / oder GM-CSF bzw. G-CSF enthalten, und in entsprechendes Gewebe oder in entsprechende (defekte) Knochenstrukturen eingebracht werden, zeigen in hohem Maße eine gesteigerte Fähigkeit, die Generierung von funktionsfähigen ausdifferenzierten Zellen des Zielgewebes zu bewirken.

Dabei wird ein weiterer Vorteil sichtbar, dass nämlich bei Defektgrößen oder auch Defektarten, die keine alleinige restitutio ad integrum bzw. Wiederherstellung des originalen Gewebes mehr ermöglichen, hierbei Füllmateralien eingemischt werden können, die dem Zielgewebe nahe kommen und somit eine quasi "Kopiervorlage" für den Remodellingprozess der in dem Applikationscocktail aktivierten Stammzellen darstellen. Die Materialien der Kopiervorlage können z.B. mineralischer Natur für den Knochen oder Dentinersatz bei Zähnen sein. Synthetische RGD Analoga, Collagenpeptide (bevorzugt vor tierischen Collagenen) können hier in der natürlichen Zusammensetzung des Knochens hinzu gemischt und vor Ort in der Wunde bzw. dem Defekt zur Gelpolymerisation gebracht werden.

Gegenstand der Erfindung ist somit ein wie in den Ansprüchen definiertes Polymerisat aus bevorzugt autologen und im wesentlichen humanem oder tierischem Blut oder Blutbesatndteilen mit allen seinen Verfalls-Bestandteilen wie zellulären Detritus, welches sich dadurch auszeichnet, dass es Stammzellen oder Knochenmarkzellen enthält, sowie mindestens eine Substanz, welche die Freisetzung, Vermehrung oder Differenzierung der Stamm- oder Knochenmarkzellen bewirkt oder fördert oder verstärkt.

Entsprechende Substanzen, welche das Wachstum und die Differenzierung der Stammzellen fördern, sind Wachstumshormone, wie HGH oder Cytokine, wie z.B. Interleukine, Interferone, TNFa oder G-CSF oder GM-CSF.

Insbesondere ist Eryrthopoietin (EPO) oder eines seine biologisch aktiven Analoga, Derivate oder Fragmente geeignet. Erythropoietin (EPO) ist ein Glykoprotein-Hormon, das die Bildung der Erythrozyten aus Vorgängerzellen im Knochenmark (Erythropoese) steuert. EPO bindet dabei an seinen Rezeptor (EPO-R), der auf allen hämatopoietischen Zellen exprimiert wird.

In den letzten Jahren wurde von diversen Autoren berichtet, dass EPO auch eine nichthämatopoietische Wirkung ausübt, und das EPO-R entsprechend auch von bestimmten nicht-hämatopoietischen Zellen exprimiert wird. So wird von einer Stimulierung durch EPO von Nervenzellen, neuronale Zellen des Gehirn und Endothelzellen berichtet, wobei dies in einigen Fällen mit einer direkten Expression des hämatopoietischen EPO Rezeptors verbunden ist.

In anderen Fällen wird das Vorhandensein eines weiteren, nicht-hämatopoietischen Rezeptors prognostiziert. Insbesondere der noch nicht sehr lange bekannten nicht-hämatopoietischen Wirkung von Erythropoietin (EPO) im Zusammenhang beispielsweise mit der angeregten Bildung und Regeneration von Endothel- und Gewebezellen wird zunehmend Bedeutung beigemessen.

So beschreibt die WO 2004/ 001023 unter anderen die Verwendung von EPO und TPO zur Anregung der Gefäßneubildung und Geweberegeneration und Verbesserung der Wundheilung, z.B. nach Operationen oder Verletzungen.

In der WO 2005/063965 wird die Verwendung von EPO zur gezielten strukturell gelenkten Regeneration von traumatisiertem Gewebe gelehrt, bei welcher nicht nur ein Endothelzellwachstum stimuliert, sondern auch die Parenchymregeneration und die Ausbildung der Wandstrukturen gefördert wird, so dass ein koordiniertes dreidimensionalen Wachstum zum Aufbau eines funktionsfähigen Gewebes, Organs oder Teilen davon von statten geht. Erythropoietin, und EPO-Derivate oder auch EPO-Mimetika scheinen bei systemischer Anwendung also geeignet zu sein, um bei Verletzungen der Haut, der Schleimhaut, bei offenen Haut- und Fleischwunden oder auch bei Hautirritationen durch Verbrennungen oder Verbrühungen die Neubildung und Regeneration des betroffenen Gewebes gezielt zu initiieren und zu steuern und letztlich die Heilung fördern und beschleunigen zu können.

Die WO 2005/070450 sowie weitere Arbeiten der betreffenden Erfinder beschreibt die Verwendung von EPO bei der Regeneration von Gefäßen und Gewebe mit eine wöchentlichen Dosis von unter 90 IU/kg Körpergewicht, unter anderem auch für den Bereich der Wundversorgung. Dadurch soll erreicht werden, dass die Blutbildung im Knochenmarkbereich weniger stimuliert wird, aber nach neuerer Lehre, wie geschildert, die Aktivierung von Endothelzellprogenitoren im Blutbereich möglich ist. Eine Aktivierung der Endothelzellvorläuferzellen im Blut aber auch im Gewebe und die Entwicklung der Endothelzellen, welche die innerste Zellschicht der Blutgefäße bilden, wurde mit einer Verbesserung der Gefäßbildung in Zusammenhang gebracht und es wird vermutet, dass dadurch auch eine Geweberegeneration ermöglicht wird. Dies konnte in der Zwischenzeit in klinischen Versuchen bei Verbrennungswunden belegt werden.

Erfindungsgemäß wurde nun gefunden, dass der durch Blutpolymerisate bereits geförderte Effekt auf die Stammzellenentwicklung und - Differenzierung weiter um etwa 10 - 50% gesteigert werden kann, wenn man die entsprechenden Polymerisate zusätzlich EPO enthalten, wobei die EPO-Dosis zwischen 50 und 500 IU/kg Körpergewicht, vorzugsweise zwischen 100 und 300 IU/kg Körpergewicht beträgt. Der Grund liegt nicht nur in der zur Verfügung gestellten 3D Struktur sondern in der überraschenden synergistischen Effekten, die entsprechend dem Stand der Lehre bisher gegenteilig interpretiert wurden. So ist es bekannt, dass höhere Schichtdicken im Tissue-Engineering nicht erreicht werden können, da der eintretende Sauerstoffmangel die Gewebebildung auf wenige Mikrometer an Schichtdicken begrenzt (zumeist 100-500µm). Zusätzlich werden hochreine Zellpopulationen hergestellt, die keine abgestorbenen Zellen enthalten sollen.

Die Gesamtheit der Kombination stellt jedoch einen überraschend positiven Effekt für die in das Blutpolymer eingegebenen Stammzellen dar, wobei die Polymerisation, die erhöhten Schichtdicken, der zelluläre Zerfall von z.B. den mononukleären Zellen und deren Aktivierung unter ischämischen Zuständen Akutphasenreaktionen an den heterogenen Stammzellen vermittelt, die in Verbindung insbesondere mit EPO einen explosiven Triggerereffekt auslöst. Werden dann noch selektive "kopierfähige" Komponenten des extrazellulären Milieus hinzugegeben, entsteht erfindungsgemäß ein echter "remodelling" Effekt der zu einer de novo Gewebebildung führt. Diese Gewebebildung kann dann auch entstehen, wenn Art und Größe des Defektes keine eigene Heilung ermöglichen würden.

Gegenstand der Erfindung ist somit ein Stammzellen oder Knochenmarkzellen aufweisendes Blutpolymerisat, welches zusätzlich Erythropoietin (EPO) in der geeigneten Dosierung enthält. Der Einsatz derartiger Polymerisate bewirkt je nach gewebespezifischer Applikation, dass die Geweberegeneration um 10 - 50 % schneller und qualitativ/funktionell verbessert erfolgt, als die herkömmlichen Methoden, die auf Einsatz von bereits spezialisierten Gewebezellen beruhen.

Die erfindungsgemäßen EPO-haltigen Stammzellenpolymerisate auf Basis von vorzugsweise autologen Blut oder Blutplasma oder Blutplättchen können zusätzliche Wachstum und Differenzierung fördernde Substanzen, insbesondere GM-CSF, G-CSF oder TNFalpha aufweisen.

Durch EPO und seine biologisch aktiven Derivate wird erfindungsgemäß in Verbindung mit einem Polymerisationsprozess von Blut / Knochenmark/Blutplättchenkonzentrat, Plasma, roter und/ oder weisser Blutkörperchenfraktion das Überleben und die Weiterentwicklung der Stammzellen oder deren Vorläuferzellen sowie eine gewebespezifische Aktivierung und im Traumafall eine spezifische Regeneration von Gewebe ermöglicht.

Der Erfindung liegt die grundsätzliche Entdeckung zugrunde, dass Blut oder Blutplasma in Verbindung mit Stammzellen eine viskose Konsistenz annimmt und die dadurch offensichtlich entstehenden 3D-Strukturen die Entwicklung der Stammzellen begünstigen. Durch Einbringung von exogenen Gelbildnern in die flüssige Mischung aus Blut/Blutplasma, Stammzellen, EPO etc. kann dieser Effekt noch verstärkt werden, wobei Gele bzw. ein Polymerisate von unterschiedlicher Festigkeit und mit guten Verarbeitungseigenschaften erzeugt werden können. Als einfacher und erfindungsgemäß sehr erfolgreicher Gelbildner haben sich beispielsweise Thrombin mit und ohne Calcium-Ionen, oder Protamin herausgestellt, welche zu der noch flüssigen Mischung gegeben werden können.

Hieraus können spezifische Applikationsverfahren Anwendung finden, indem z.B. das Stammzellen enthaltende Gemisch in einer Spritze über eine forcierte Mischung mit einer die "Kopiervorlagen" enthaltende 2. Spritze in eine einzige Applikationskanüle noch flüssig überführt wird und erst am Anwendungsort polymerisiert.

Einen ausreichenden Polymerisationseffekt im oben diskutierten Sinn, der sich günstig auf die Entwicklung der Stammzellen oder ihrer Vorläuferzellen auswirkt, erhält man auch durch Zugabe von Zelldetritus zu der zu polymerisierenden Mischung. Zelldetritus entsteht durch aus zellhaltigen Proben durch Absterben von Zellen, wie mononuklelären Zellen, roten und weißen Blutkörperchen (Leukozyten), Blutblättchen oder Stammzellen, Vorläuferzellen, Fibroblasten, Endothelzellen, Bindegewebszellen, Knorpelzellen und Makrophagen aufgrund von Mangelversorgung, wobei vorteilhafterweise Stoffe freigesetzt werden, welche vorteilhafte Eigenschaften für die Polymerisation bzw. die Qualität des Polymerisats aufweisen. So werden auch endogen Calcium-Ionen freigesetzt, die eine Zugabe von exogenem Calcium ganz oder teilweise erübrigen. Vorteilhafterweise wird Zelldetritus autologen Ursprungs zu den zu polymerisierenden Mischungen gegeben.

Gegenstand der Erfindung ist somit auch ein entsprechendes bluthaltiges Polymerisat, welches endogene oder exogene Substanzen enthält, die fixierbare und konformative Strukturen der Stammzellen und damit deren Entwicklung fördern. Derartige Substanzen können Thrombin, Calcium-Ionen, Zelldetritus verschiedenartiger, vorzugsweiser autologer Zellen, biologische Kollagene oder deren Fragmente, Bestandteile der extrazellulären Matrix, Fibrin, Fibrinkleber oder andere gelbildenden Substanzen sein.

Die Polymerisation kann ersatzweise oder zusätzlich durch andere natürliche oder synthetische Polymerbildner bewirkt werden, wie beispielsweise gelbildende quellfähige Polysaccharide, wie Hydroxyalkycellulosen und / oder Caboxyalkylcellulosen oder synthetische Plolmerbildner auf Basis von Acrylaten, wie z.B. (Poly)Methacrylat, (Poly)Methylmethacrylat, Polyacrylamid, (Poly)ethoxyethylmethacrylat.

Ferner wurde festgestellt, dass durch Zugabe von lyophilisiertem Blut in die zu polymerisierende Mischung die Polymerisationsstabilität des Polymerisats sowie die Eigenschaft desselben in Bezug auf seinen Gewebe regenerierenden Effekt verbessert bzw. verstärkt wird. Zusätzlich können klebende Proteine auf der Basis des Jakobsmuschel in nativer order synthetischer Form beigemischt werden. Ebenso können Serotonine hinzugegeben werden, diese stimulieren nicht nur neuronale Progenitoren sondern unterstützen eine klebende Wirkung.

Mit Hilfe von Thrombin, Calcium-Ionen und / oder Zelldetritus und /oder anderen geeigneten Gelbildnern und / oder lyophilisierten Blut stehen mehrere Möglichkeiten zur Verfügung, die Polymerisationseigenschaften der Mischung aus Blut/Blutplasma und Stammzellen zu variieren und zu verändern, und hierdurch überraschenderweise auf die Stammzellentwicklung und -Differenzierung Einfluss zu nehmen.

Erfindungsgemäß brauchen die Stammzellen nicht eigens isoliert zu werden. Es ist auch ausreichend, direkt Knochenmark eines Individuums zu verwenden und dieses mit vorzugsweise autologem Blut oder Blutplasma zu vermischen, durch Zentrifugation, Filtration oder Sedimentation bei Bedarf in Bezug auf die zellulären Komponenten aufzukonzentrieren und mit Thrombin, Calcium-Ionen und / oder Zelldetritus, Matrizes (Mineralien, Peptiden, Zuckern, Lipiden und Kombinationen davon) und vorzugsweise EPO zu versetzten und zur Gelbildung zu bringen. Ein derartiges Gel kann direkt für die unterschiedlichsten Applikationen eingesetzt werden.

So ist Gegenstand der Erfindung ein entsprechendes Polymerisat, welches zur Regeneration von Gewebe oder Knochen, insbesondere von traumatisiertem Gewebe und / oder unter anderem Knochen in vivo eingesetzt werden kann.

Insbesondere ist Gegenstand der Erfindung ein entsprechendes Polymerisat zur Verwendung zur Regeneration von traumatisierten Geweben oder Knochenstrukturen, wobei das Polymerisat vorgesehen ist, in den betreffenden traumatisierten Bereich oder in dessen unmittelbare Nähe eingebracht, oder zur Auffüllung von Defekten verwendet zu werden.

Es hat sich gezeigt, dass bei Knochendefekten ein solches erfindungsgemäßes Polymerisat besonders vorteilhaft in Verbindung mit Knochenersatzmaterialien eingesetzt werden kann. Hierbei können das Gemisch kurz vor Eintritt der Polymerisation und das noch nicht auspolymerisierte Knochenersatzmaterial gleichzeitig in den Knochendefekt gebracht, und dort vermischt werden, um danach zu polymerisieren. Besonders vorteilhaft ist dies für z.B. nanokristallines Hydroxylapatit oder anderes nanokristallines mineralisches oder biologisches Material (Proteine, Peptide, Lipide, Zucker, Kunststoffe), das entweder in einer trockenen oder hydrierten Form vorliegt. Durch diese pulvrige oder pastöse Materialeigenschaft ist die Besiedelbarkeit mit Stammzellen eingeschränkt oder sogar zum Nachteil der biologischen Materialien. So wurde gezeigt, dass derartiges Material (z.B. "Ostim" ) nicht besiedelbar ist. Erfindungsgemäß ist es trotzdem und überraschenderweise möglich, eine hohe Stammzelldichte in dem als nicht besiedelbar geltenden nanokristallinen Material zu erreichen. Nach einer in vivo Implantation führt dies zu einer Beschleunigung des Knochenaufbaues um ca. 50 % bereits ohne die Zugabe von EPO. Bei einem zusätzlichen Miteinbringen von EPO in topischer Form kann der Aufbau des Knochens zusätzlich um 10-20% beschleunigt werden. Alternativ kann auch des Scaffold Material als Pulver bereits mit EPO, dessen Derivaten, oder Peptidfragmenten, versetzt werden. Dies hat den Vorteil der bessern Lagerfähigkeit.

Alternativ kann, z.B. bei der Verwendung von zwei Applikationsbehältern in Form von Spritzen das EPO in lyophilisierter Form in der Spritze vorbereitet werden, die das Blut, Knochemark-Stammzellgemisch aufnimmt. Auf dieser Seite (Spritze 2) können ebenso weiter Matrix (Scaffold) Komponenten autologen Ursprungs (lyophilisiertes Blut oder Plasma, Fibrin, Thrombin) sowie synthetischen Ursprungs (Peptidfragmenten von Matrixproteinen, selbst-aggregierende Peptidstrukturen (RADA), Phosphatidylcholin, Sphingolipide, Lecithin, HDL (High Density Lipoproteine), sowie Glucose, Glucosamine, Glucosaminsulfate, Hyaluronsäure, Chitosan, Seidenproteine, Kleberproteine der Jakobsmuschel, Collagene und deren Fragmente sowie Peptide vorliegen.

Dieses Applikationsverfahren ermöglicht es, dass die Stammzellen optimal verteilt werden und innerhalb der z.B. Knochenersatzmatrix eine heterogene "Scaffold"-Struktur erhalten, in welche Knochengewebe von außen über Eintrittsstraßen direkt hineinwachsen und diese sukzessive ersetzen kann. Die hierdurch entstehende inter-konnektierenden Porositäten sind somit *ab initio* mit Stammzellen in gelartigen Strukturen durchsetzt. Hierdurch entstehen optimale Wachstumsbedingungen trotz sehr hoher Schichtdicken.

Entgegen zur konventionellen Lehre führt die Zugabe von EPO,GM-CSF oder G-CSF, insbesondere aber EPO in dieser Situation zu einer besonders effizienten Stimulierung von Stammzellen bzw. Progenitoren in optimierten Milieus. Hierdurch ist es möglich, auf entsprechende Expansionsverfahren von Progenitoren außerhalb des Applikationsortes (Praxis, Operationsraum) zu verzichten und zeitgleich zum eigentlichen operativen Eingriff (intraoperativ) die Stammzelltherapie durchzuführen. Dies hat wesentliche regulatorische und ökonomische Vorteile (Kostensenkung), bedeutet eine Risikominimierung und eine wesentliche Qualitätsverbesserung.

Bei besonders großen Defekten kann eine Vorexpansion in dem Stammzellgel jedoch vorteilhaft sein. Erfindungsgemäß kann diese Kultivierung jedoch dann sofort in dem 3D Blutgel oder auch Plasmagel erfolgen. Zu diesem Zweck kann die Polymerisation auch in vitro ausgelöst werden, um dadurch das Wundmilieu in vitro zu schaffen, das ideale Wachstumsbedingungen für die Stammzellen / Progenitoren in einem parakrinen Stimulationsmilieu unter Sauerstoffmangel wegen der erhöhten Schichtdicken von mehreren Millimetern Dicke bis zu mehreren Zentimetern Dicke im Durchmesser auszulösen. Bei der intraoperativen Variante genügen bereits wenige Sekunden bis einige Minuten, um die notwendige Stimulationsprozesse der Stammzellen simultan auszulösen (IL-6, EPO, GM-CSF, G-CSF, Matrix).

Gegenstand der Erfindung ist somit die Verwendung eines diesbezüglichen Polymerisats in Verbindung mit einem Knochenersatzmaterial insbesondere zur Regeneration und zum Wiederaufbau von defekten, traumatisierten oder erkrankten Knochenstrukturen oder Knochengeweben in vitro, in vivo oder ex vivo.

Hierbei sollten die mineralischen Komponenten des Knochenersatzmaterials geeignet sein, das Auffüllen des Knochendefektes zu unterstützen. Als geeignete Knochenersatzmaterialen sind zum Beispiel Hydroxylapatit, Tricalciumphosphat und ähnliches zu nennen.

Die erfindungsgemäßen Polymerisate eigenen sich für die mannigfaltigsten medizinischen Verwendungen, bei denen Gewebe- oder Knochendefekte durch Traumatisierung oder Erkrankung entstanden sind. Insbesondere können sie auch im Dentalbereich bei Zahn- und Kieferdefekten eingesetzt werden, aber auch im topischen Bereich bei Verletzungen der Haut oder Schleimhaut.

Gegenstand der Erfindung ist ferner ein Verfahren zur Herstellung eines entsprechenden Polymerisats umfassend humanes oder tierisches Blut sowie humane oder tierische Stamm- oder Knochenmarkzellen, wobei man besagte Komponenten im flüssigen Zustand miteinander vermischt und dieses Gemisch mittels eines Gelbildners, insbesondere durch Zusatz von Calcium-Ionen, Thrombin, Fibrin oder Bestandteilen der extrazellulären Matrix biologischen Ursprungs oder Zelldetritus und gegebenenfalls zusätzlich lyophilisiertem Blut polymerisiert bzw. verfestigt, wobei man in einer besonderen Ausführungsform in das flüssige Gemisch vor Polymerisierung eine oder mehrere Substanzen hinzugibt, welche die Freisetzung, Vermehrung oder Differenzierung der Stamm- oder Knochenmarkzellen bewirkt oder fördert. Gegenstand der Erfindung ist ferner ein wie in den Ansprüchen definiertes Verfahren zur Regeneration von Gewebe oder Knochenstrukturen in vitro oder ex vivo umfassend die Schritte:
(i) Bereitstellung von isolierten Stammzellen oder Knochenmark
(ii) Einbringen besagter Zellen in eine Blut-, Blutplättchen oder Blutplasmaprobe
(iii) Polymerisierung der Probe aus (ii) und
(iv) Züchten der Zellen in einem geeigneten Milieu, welches das Wachstum und die Differenzierung der Zellen zu dem gewünschten Gewebe initiiert und / oder fördert.

Gegenstand der Offenbarung ist außerdem ein Verfahren zur Regeneration von Gewebe oder Knochenstrukturen umfassend die Schritte:
(i) Bereitstellung von isolierten Stammzellen oder Knochenmark
(ii) Einbringen besagter Zellen in eine Blut- oder Blutplasmaprobe, oder direkte Verwendung von Knochenmark und Vermischung mit EPO, GCSF, GM-CSF
(iii) Polymerisierung der Probe aus (ii) mit z.B. Ca++ oder Prothrombin, Protaminund ggf. mit weiteren Faktoren wie "Kopiermateralien" und Differenzierungsfaktoren synthetischen oder natürlichen Ursprungs.
(iv) Einbringen der Probe unmittelbar in den verletzten, erkrankten, defekten oder degenerierten Bereich des Körpers.

Die oben erwähnten Differenzierungsfaktoren können TGFbeta oder Parathormon (Knorpelbildung), oder Vitamin C (für eine Induktion einer neuronalen Sprossung) sein.

Als wichtige Ergänzung zur Verbesserung des "Tissue Engineering" werden Fragmente oder Partikel des Zielgewebes mit integriert. Dies haben idealerweise eine Dicke von ca. 100-200 µm und einen Durchmesser von ca. 200-300 µm. Größere oder kleinere Fragmente sind möglich. Die Zerkleinerung erfolgt idealerweise mit einem Skalpell oder scharfen Messer. Der Vorteil ist, dass dieser Vorbereitungsprozess nur sehr wenig Zeit in Anspruch nimmt. Dieser gesamte Prozess kann daher zeitgleich in einer bevorzugten Form zur eigentlichen Operation gemacht werden.

Alternativ zu dem kompletten Gemisch der im Knochenmark enthaltenen Zellen könnten auch isolierte Stammzellen oder Progenitorpopulationen wie CD31, CD71 und CD 134, und CD90 positive Zellen eingesetzt werden.

Gegenstand der Offenbarung sind insbesondere solche Verfahren in vivo, in vitro und ex vivo, bei denen die Polymerisierung der Blut oder Blutplasmaprobe durch Zugabe von Calcium-Ionen und / oder einer natürlichen oder synthetischen Gel bildenden Substanz, und /oder Zelldetritus erfolgt.

Gegenstand sind weiterhin solche Verfahren in vivo, in vitro und ex vivo, bei denen Wachstumsfaktoren und / oder Hormone und / oder das Wachstum oder die Differenzierung fördernde Substanzen und / oder Zellen hinzugeben werden, insbesondere ausgewählt aus der Gruppe bestehend aus EPO, GM-CSF, G-CSF, GH, Bindegewebszellen, Fibroblasten, Makrophagen, Zelldetritus.

Die folgenden Beispiele sollen die Erfindung weiter erläutern, ohne sie einzuschränken. Insbesondere sind die genannten speziellen Verfahrensschritte, Verfahrensparameter, Stoffe, Gewebeproben und Anwendungen nicht limitierend und können durch andere gleichwirkende Verfahrensschritte, Verfahrensparameter, Stoffe, Gewebeproben und Anwendungen ersetzt werden, wenn der Fachmann hierfür ohne weiteres einen Anlass sieht.

Die Erfindung wird durch die Ansprüche definiert.

### Beispiel 1:

### Therapie der Nervenverletzung insbesondere Querschnittverletzungen.

Bei den akuten Querschnittverletzungen muss eine Dekompression des in Mitleidenschaft gezogenen Nervenbereiches durchgeführt werden.

Erfindungsgemäß wird hierbei Erythropoietin in Verbindung mit Knochenmark und Blut versetzt und auf den Wundbereich wie eine Salbe bzw. Gel aufgetragen.

Idealerweise hat das Gel eine Schichtdicke von ca. 1-2 mm und besitzt ca. 100 000 Zellen. Bei größeren Defekten im Spinalkanal kann das Knochenmark mit lyophilisierten Blut oder alternativ mit einem zerkleinerten Kollagenschwamm vermischt werden. Dies fördert die Ausbildung einer größeren Masse und einer Leitstruktur. In diese Masse kann ebenso eine Blutplättchenkonzentrat native oder gefriergetrocknet hineingegeben werden. Das ganze wird zeitgleich mit Vitamin C (10-20 mg) versetzt.

### Beispiel 2:

### Knochenaufbau in Verbindung mit Mineralien

Eine der wesentlichen Vorteile der Erfindung liegt darin, dass fertige Produkte wie z.B. Knochenersatzmaterialien in wässriger Lösung, die im wesentlichen nicht besiedelbar sind, trotzdem in einer sehr effektiven Weise mit Stammzellen versehen werden können.

Hierzu werden in einer Tandemspritzensystem in einer ersten Spritze des konventionelle Knochenersatzmaterial und in einer zweiten Spritze das Blut -Stammzellgemisch mit und ohne EPO, mit oder ohne Calcium-Ionen, Zeldetritus, etc. verwendet. Im einzelnen wird in einer leeren Spritze eine Lyophilisat von EPO (z.B. 10 000 IU für einen Mensch mit 70 KG Gewicht), zusätzlich Ca++ (1 mg in kristalliner Form oder 1 mol/l) hinterlegt. In diese Spritze wird 1 - 2 ml Knochenmark eingesaugt sowie zusätzlich 1 ml Blutplättchen-Konzentrat. Die Mischung dieser zweiten Spritze wird dann parallel über eine forcierte Mischungsschraube aus dem Tandemspritzensystem zeitglich mit dem in der ersten Spritze befindlichen noch verformbaren Knochenersatzmaterial in einen Knochendefekt appliziert und dabei vor Ort gemischt und zu Polymerisation gebracht.

Die zweite Spritze kann zusätzlich synthetische oder biologische Kollagene oder deren Fragmente, Bestandteile extrazellulärer Matrix, oder andere Stoffe wie z.B. RGD Peptide enthalten. Zusätzlich können BMP oder Blutplättchen oder Blutkonzentrate in lyophilisierter oder nativer Form als Konzentrat hinzu gemischt werden.

### Beispiel 3:

### Auffüllung einer Knochenzyste:

Für die Herstelllung einer pastösen und flexiblen Masse zur Auffüllung von Hohlräumen wird wie folgt vorgegangen: Es werden ca. 10 ml Knochenmark in ein Röhrchen / Spritze gegeben in dem sich lyophilisiertes Erythropoetin, lyophilisiertes Blutplasma und nanokristallines Hydroxylapatit oder Tricalciumphosphat befindet.

Es entsteht innerhalb kurzer Zeit eine hochpastöse klebrig Masse, die in den Defektbereich des Knochens mit einem Spatel oder über eine Kanüle eingeben wird.

Ferner werden synthetischen Komponenten der extrazellulären Matrix wie Fibronectin oder Kollagenen hinzugegeben, wodurch beschleunigt Wachstumszonen für die Integration der zellulären Systeme entstehen bzw. diese verbessern.

### Beispiel 4:

### Regeneration von Herzmuskelgewebe:

Es wird ca. 500 ml peripheres Blut abgenommen und bei 50g für 5 min zentrifugiert. Danach wird der Überstand nochmals bei 800g für 5 min zentrifugiert und das Pellet mit dem vorherigen Pellet zusammengeführt. Alle so erhaltenen Zellen aus dem peripheren Blut werden in einer Spritze die 250 Units / kg Köpergewicht EPO aufgenommen und nach einer Inkubationszeit von 5 min und Aktivierung der EPO Rezeptoren den Stammzellen implantationsreif. Alternativ oder in Kombination können 5-10 ml von Knochenmark in die Spritze mit dem lyophilisiertem EPO gegeben werden.

In analoger Weise wird vorgegangen um nach Muskelfaserrissen Stammzellen in den verletzten Bereich zu applizieren. Hierfür werden ca. 1-2 ml pro Injektionsort verwendet.

### Beispiel 5:

### Regeneration von Brustdrüsengewebe:

2-3 ml Fettgewebe aus dem Kniegelenksbereich wird mit abzentrifugiertem Knochenmark versetzt und mit 250 Units / KG /KGW topisch appliziert. Nach einer Inkubationszeit von ca. 10 sec bis 5 min sind die Stammzellen injektionsbereit. Die Injektionslösung kann lyphilisiertes Thrombin und Ca++ enthalten. In analoger Weise wird das Stammzellgemisch für die Anwendung zum Aufbau von subkutanem Fettegewebe und zur Faltenreduktion und Verjüngung der Haut eingesetzt. Die Stammzellen aus dem Knochenmark (aus 10 ml) oder periphren Blut (aus 100 ml), werden nach Zentrifugation mit Thrombin und / oder Ca++ versetzt und subkutan mit einem Volumen von ca. 500 µl pro Injektionsort appliziert. Dieser Vorgang kann alle 3-4 Tage oder wöchentlich wiederholt werden.

### Beispiel 6:

### Regeneration von Zwischenwirbelscheiben (Bandscheiben)

Bei den akuten oder chronischen Bandscheibenverletzungen muss eine Dekompression des in Mitleidenschaft gezogenen Nervenbereiches durchgeführt werden. Bei Resektion eines Sequesters wird dieser Nervenbereich entlastet. Aus diesem Sequestermaterial werde durch mechanische Zerkleinerung kleinste Gewebefragmente hergestellt. Diese (±0.5 ml) werden mit dem Knochenmark ( 2 ml) entweder in einer nativen oder aufkonzentrierten Form versetzt und zugleich lyophilisiertes EPO, GM-CSF oder G-CSF in lyophiliisierter Form entsprechend den üblichen Dosierungsempfehlungen in Bezug auf das Körpergewicht hinzugegeben. Hier erfolgt jedoch keine systemische sondern bevorzugt eine topische Applikation. Das ganze Gemisch kann mit 1-2 ml Blutplättchenkonzentrat bevorzug in lyophilisierter Form versetzt werden.

Erfindungsgemäß wird hierbei Erythropoietin in Verbindung mit Knochenmark und/oder Blut versetzt und auf den Bandscheibenbereich wie Gel mittels einer sterilen Spritze injiziert.

Idealerweise weist das Gel ca. 100000 -1000 000 Zellen, mehr oder weniger ist jedoch möglich.

Bei größeren Defekten im Bandscheibenbereich kann das Knochenmark mit lyophilisierten Blut oder alternativ mit einem zerkleinerten Kollagenschwamm vermischt werden. Dies fördert die Ausbildung einer gröeren Masse und einer Leitstruktur. In diese Masse kann ebenso eine Blutplättchenkonzentrat native oder gefriergetrocknet hineingegeben werden. Das Ganze wird zeitgleich mit Vitamin C (10-20 mg) versetzt.

### Beispiel 7:

### Regeneration von Knorpelgewebe

Bei den akuten oder chronischen Knorpelverletzungen und Arthrosen wird mittels eines weichen z.B. Silikonschlauchs in einen gering eröffneten Gelenkbereich das Polymerisat kurz vor der Verfestigung eingeführt, so dass die Polymerisation vor Ort erfolgen kann. Bei Resektion von Sequestern können diese durch mechanische Zerkleinerung in kleinste Gewebefragmente gewandelt werden. Diese (±0.5 ml) werden mit dem Knochenmark ( 2 ml) entweder in einer nativen oder aufkonzentrierten Form versetzt und zugleich lyophilisiertes EPO, GM-CSF oder G-CSF in lyophiliisierter Form entsprechend den üblichen Dosierungsempfehlungen in Bezug auf das Körpergewicht hinzugegeben. Hier erfolgt jedoch keine systemische sondern bevorzugt eine topische Applikation. Das ganze Gemisch kann mit 1-2 ml Blutplättchenkonzentrat bevorzug in lyophilisierter Form versetzt werden.

Erfindungsgemäß wird hierbei Erythropoietin in Verbindung mit Knochenmark und/oder Blut versetzt und auf den Bandscheibenbereich wie Gel mittels einer sterilen Spritze injiziert. Idealerweise hat das Gel besitzt ca. 100000 -1000 000 Zellen, mehr oder weniger ist jedoch möglich.

Bei größeren Defekten im Knorpelbereich kann das Knochenmark mit lyophilisierten Blut oder alternativ mit einem zerkleinerten Kollagenschwamm vermischt werden. Dies fördert die Ausbildung einer größeren Masse und einer Leitstruktur. In diese Masse kann ebenso eine Blutplättchenkonzentrat native oder gefriergetrocknet hineingegeben werden. Das Ganze wird zeitglich mit Vitamin C (10-20 mg) versetzt. Hierdurch können auch sehr große Gelenkflächen versorgt werden. In den oberen Gelenkbereichen zum Gelenkspalt hin wird vermehrt das Blutplättchenkonzentrat geschichtet eingesetzt. In bestimmten Fällen kann TGFbeta und / oder Parathormon zur Förderung der Knorpelbildung eingesetzt werden.

### Beispiel 8:

### Regeneration von Sehnengewebe und Meniskus

Bei verletzten Sehengewebe (z.B. Achillessehne bei Mensch und Pferd) oder Meniskus wird aufkonzentriertes Knochenmark versetzt mit EPO und GCSF oder GM-CSF in den verletzten Bereich injiziert. Dehiszenzen werden idealerweise mittels Naht in üblicher Weise approximiert. Gewebefragmente aus Abrissbereichen werden hergestellt, in das Polymeristionsgel gebracht und mit diesen in den Dehiszenzbereich und darum re-injiziert.

In analoger Weise erfolgt die Regeneration von Innenohrknöchelchen oder die der Retina.

### Beispiel 9:

### Verbesserung von Implantatoberflächen zur Vermeidung von Kapselfibrosen bei Brutsprothesen:

Eines der großen Probleme bei der Implantation von Brustprothesen besteht darin, dass sich eine Kapselfibrose um das Implantat entwickelt. Erfindungsgemäß wird eine Mikrostruktur auf die Implantatoberfläche aufgebracht, die ein Einwachsverhalten von Stammzellen auf diesen Oberflächen ermöglicht. Diese Mikrostruktur besitzt Kavitäten mit einem unterem Durchmesser von idealerweise 4-5 µm und einem Durchmesser für die größeren Kavitäten von 25-250 µm. Diese Kavitätenstruktur kann idealerweise mit konnektierenden Kanalstrukturen verbunden werden, die die Selbstorganisation eines vaskulären Bettes ermöglichen. In den Kavitäten wird eine direkte Ansiedlung von Knochenmark idealerweise nach einer frischen Entnahme aus den Knochenmark durchgeführt. Zur Aufkonzentrierung der Zellen kann eine Zentrifugation bei ca. 30-50 g eine schonende Anreicherung ermöglichen. Zur Auslösung der Polymerisation und zum Initiieren der Gewebeneubildung wird Thrombin zugesetzt.

Die Zellen aus dem Knochenmark werden mit Blut vermischt durch Zugabe von Thrombin in den Mikrostrukturen zur Gelbildung und Polymerisation gebracht. Das Thrombingemisch / Stammzellgemisch in Verbindung mit Blut hat eine besonders regenerationsfreundliche Wirkung. Hierdurch werden in Verbindung mit dem regionalen Wundbereich an der Implantationsstelle Wachstumsprozesse ausgelöst, die zu einer um ca. 50% reduzierten Fibrose führen. In der Verbindung mit der vorteilhafterweise topischen Anwendung von Erythropoietin entsteht ein regionaler Stimulationseffekt, der zu einer direkten Aktivierung eingebrachter sowie auch ortständiger Progenitoren führt. Hierzu zählen adipogene als auch Drüsenprogenitoren. Die Oberflächenmarker sind CD 90, SCA1, CD 71 werden auf diesen Progenitioren gefunden. Die Kapselfibrose spielt auch bei der Implantation von Netzen nach Hernien, oder bei der Schließung von Bauchwanddefekten eine bedeutende den Heilungsverlauf komplizierende Rolle.

In analoger Weise werden weitere Implantate mikrostrukturierten Oberflächen mit dem vor Ort polymerisierenden Stammzellblutgel mit oder ohne EPO / Derivaten / Analoga versehen.

In analoger Weise erfolgt die Behandlung von Hüftprothesen oder andere Gelenkprothesen.

### Beispiel 10:

### Dentale Implantate:

Die Regeneration des Dentins nach einer Wurzelbehandlung kann erfindungsgemäß durch Einbringen eines nanostrukturierten Stammzell-Blutgels mit Hyroxylapatit oder Tricalciumphosphat mit oder ohne EPO wie auch mit oder ohne Stammzellen erreicht werden. Die Granulatgröße bzw. Mineralgröße ist hierbei idealerweise 5-100 µm, wobei Vergrößerungen nach oben Verschlechterungen der Fließeigenschaften in dem Bohrkanal bedeuten.

### Beispiel 11:

In analoger Weise wie in den oben genannten Beispielen beschrieben, können andere Implantate behandelt werden, wie z.B.: Herzschrittmacher, Bauchwandnetze, Trachealersatz, Gefäßersatz, oder Herzklappenersatz.

### Beispiel 12:

### Regeneration von Verbrennungswunden, Dekubitus oder diabetischen Ulcus, infizierten Wunden

Bei den akuten oder chronischen Hautverletzungen wird im Anschluss an eine Säuberung des Wundgrundes eine Stammzellgel wie folgt hergestellt und topisch positioniert.

Aus Bereichen nicht verletzter Haut werden durch mechanische Zerkleinerung kleinste Hautfragmente wie oben prinzipiell beschrieben hergestellt. Diese (±0.5 ml) werden mit dem Knochenmark (2 ml) entweder in einer nativen oder aufkonzentrierten Form versetzt und zugleich lyophilisiertes EPO, GM-CSF oder G-CSF entsprechend den üblichen Dosierungsempfehlungen in Bezug auf das Körpergewicht hinzugegeben. Hier erfolgt jedoch keine systemische sondern bevorzugt eine topische Applikation. Das ganze Gemisch kann mit 1-2 ml Blutplättchenkonzentrat bevorzugt in lyophilisierter Form versetzt werden.

Erfindungsgemäß wird hierbei Erythropoietin in Verbindung mit Knochenmark und/oder Blut versetzt und auf den Wundbereich wie Gel mittels einer sterilen Spritze oder einem Spatel aufgegeben. Idealerweise hat das Gel besitzt ca. 100000 -1000 000 Zellen / 10 cm², mehr oder weniger ist jedoch möglich.

Bei größeren Defekten kann das Knochenmark mit lyophilisierten Blutoder alternativ mit einem zerkleinerten Kollagenschwamm vermischt werden. Dies fördert die Ausbildung einer größeren Masse und einer Leitstruktur. In diese Masse kann ebenso eine Blutplättchenkonzentrat native oder gefriergetrocknet hineingegeben werden. Das ganze wird zeitgleich mit Vitamin C (10-20 mg) versetzt.

## Patentansprüche

1. Zur Regeneration von traumatisiertem Gewebe geeignetes Polymerisat umfassend humanes oder tierisches Blut, Blutplasma oder Blutplättchenkonzentrat, **dadurch gekennzeichnet, dass** es enthält:
(i) autologe Stammzellen oder Knochenmarkzellen,
(ii) Erythropoietin (EPO) in einer Dosis zwischen 50 und 500 IU/kg Körpergewicht;
(iii) zusätzliches lyophilisertes Blut oder Blutplasma und
(iv) ein natürliches oder synthetisches Polymer und /oder Calcium-Ionen und / oder Thrombin und / oder Protamin.

2. Polymerisat nach Anspruch 1, **dadurch gekennzeichnet, dass** das Polymer Fibrin oder ein Kleber auf Basis von Fibrin ist.

3. Polymerisat nach Anspruch 1 oder 2, **dadurch gekennzeichnet, dass** es Zelldetritus und / oder weitere autologe oder heterologe Zellen aus dem Zielgewebe enthält, ausgewählt aus der Gruppe bestehend aus Fibroblasten, Endothelzellen, Bindegewebszellen, Knorpelzellen und Makrophagen.

4. Polymerisat nach einem der Ansprüche 1 - 3 zur topischen Verwendung bei der Regeneration von traumatisiertem Gewebe oder Knochen in vivo.

5. Polymerisat nach Anspruch 4 zur Verwendung bei der topischen Behandlung verletzter Haut.

6. Polymerisat nach Anspruch 4zur topischen Verwendung bei der Regeneration von traumatisierten Geweben oder Knochenstrukturen, wobei das Polymerisat vorgesehen ist, in den betreffenden traumatisierten Bereich oder in dessen unmittelbare Nähe eingebracht zu werden.

7. Polymerisat nach Anspruch 6, wobei zusätzlich ein Knochenersatzmaterial appliziert wird.

8. Verwendung eines Polymerisats nach einem der Ansprüche 1 - 3 zur Regeneration von traumatisiertem Gewebe oder traumatisierten oder defekten Knochenstrukturen in vitro oder ex vivo.

9. Verwendung nach Anspruch 8, zur Regeneration von Knochendefekten unter Zusatz von mineralischen Komponenten, die das Auffüllen des Knochendefektes unterstützen.

10. Verfahren zur Regeneration von Gewebe oder Knochenstrukturen in vitro oder ex vivo umfassend die Schritte:
(i) Bereitstellung von isolierten Stammzellen oder Knochenmarkzellen
(ii) Einbringen besagter Zellen in eine Blut- oder Blutplasmaprobe oder eines Blutplättchenkonzentrats,
(iii) Einbringen von EPO in besagte Probe
(iv) Hinzufügen von zusätzlichem lyophilisiertem Blut oder Blutplasma
(v) Polymerisierung der Probe aus (iv) und
(vi) Züchten der Zellen in einem geeigneten Milieu, welches das Wachstum und die Differenzierung der Zellen zu dem gewünschten Gewebe initiiert und / oder fördert.

11. Verfahren nach Anspruch 10, **dadurch gekennzeichnet, dass** eine oder mehrere Substanzen, Faktoren oder Zellen hinzugegeben werden, welche ausgewählt sind aus der Gruppe bestehend aus GM-CSF, G-CSF, GH, Bindegewebszellen, Fibroblasten, Makrophagen, und Zelldetritus.

## Claims

1. Polymerisate which is suitable for the regeneration of traumatised tissue, comprising human or animal blood, blood plasma or blood platelet concentrate, **characterised in that** it comprises:
(i) autologous stem cells or bone marrow cells,
(ii) erythropoietin (EPO) in a dose between 50 and 500 IU/kg of body weight;
(iii) additional lyophilised blood or blood plasma and
(iv) a natural or synthetic polymer and/or calcium ions and/or thrombin and/or protamine.

2. Polymerisate according to Claim 1, **characterised in that** the polymer is fibrin or an adhesive based on fibrin.

3. Polymerisate according to Claim 1 or 2, **characterised in that** it comprises cell detritus and/or further autologous or heterologous cells from the target tissue, selected from the group consisting of fibroblasts, endothelial cells, connective tissue cells, cartilage cells and macrophages.

4. Polymerisate according to one of Claims 1 - 3 for topical use in the regeneration of traumatised tissue or bones in vivo.

5. Polymerisate according to Claim 4 for use in the topical treatment of damaged skin.

6. Polymerisate according to Claim 4 for topical use in the regeneration of traumatised tissues or bone structures, where the polymer is intended to be introduced into the traumatised region in question or into its immediate vicinity.

7. Polymerisate according to Claim 6, where a bone replacement material is additionally applied.

8. Use of a polymerisate according to one of Claims 1 - 3 for the regeneration of traumatised tissue or traumatised or defective bone structures in vitro or ex vivo.

9. Use according to Claim 8 for the regeneration of bone defects with addition of mineral components which support the filling of the bone defect.

10. Method for the regeneration of tissue or bone structures in vivo or ex vivo, comprising the steps:
(i) provision of isolated stem cells or bone marrow cells,
(ii) introduction of said cells into a blood or blood plasma sample or a blood platelet concentrate,
(iii) introduction of EPO into said sample,
(iv) addition of additional lyophilised blood or blood plasma,
(v) polymerisation of the sample from (iv) and
(vi) cultivation of the cells in a suitable medium which initiates and/or promotes growth and differentiation of the cells to give the desired tissue.

11. Method according to Claim 10, **characterised in that** one or more substances, factors or cells selected from the group consisting of GM-CSF, G-CSF, GH, connective tissue cells, fibroblasts, macrophages and cell detritus are added.

## Revendications

1. Polymérisat qui est convenable pour la régénération de tissu traumatisé, comprenant du sang humain ou animal, du plasma sanguin ou du concentré de plaquettes sanguines, **caractérisé en ce qu'**il comprend :
(i) des cellules souches autologues ou des cellules de moelle osseuse,
(ii) de l'érythropoïétine (EPO) selon une dose comprise entre 50 et 500 UI/kg de poids corporel ;
(iii) du sang ou du plasma sanguin lyophilisé supplémentaire et
(iv) un polymère naturel ou synthétique et/ou des ions calcium et/ou de la thrombine et/ou de la protamine.

2. Polymérisat selon la revendication 1, **caractérisé en ce que** le polymère est de la fibrine ou un adhésif à base de fibrine.

3. Polymérisat selon la revendication 1 ou 2, **caractérisé en ce qu'**il comprend des débris cellulaires et/ou d'autres cellules autologues ou hétérologues issues du tissu cible, choisies dans le groupe constitué par les fibroblastes, les cellules endothéliales, les cellules du tissu conjonctif, les cellules du cartilage et les macrophages.

4. Polymérisat selon l'une des revendications 1 - 3, pour une utilisation topique dans la régénération de tissu ou d'os traumatisé *in vivo.*

5. Polymérisat selon la revendication 4, pour une utilisation dans le traitement topique de peau endommagée.

6. Polymérisat selon la revendication 4, pour une utilisation topique dans la régénération de structures osseuses ou de tissus traumatisés, où le polymère est prévu pour être introduit dans la région traumatisée en question ou dans son environnement immédiat.

7. Polymérisat selon la revendication 6, où un matériau de remplacement osseux est en outre appliqué.

8. Utilisation d'un polymérisat selon l'une des revendications 1 - 3, pour la régénération de tissu traumatisé ou de structures osseuses traumatisées ou défectueuses *in vitro* ou *ex vivo.*

9. Utilisation selon la revendication 8, pour la régénération de défauts osseux avec addition de composants minéraux qui supportent le remplissage du défaut osseux.

10. Méthode de régénération de tissu ou de structures osseuses *in vivo* ou *ex vivo,* comprenant les étapes :
(i) de fourniture de cellules souches ou de cellules de moelle osseuse isolées,
(ii) d'introduction desdites cellules dans un échantillon de sang ou de plasma sanguin ou un concentré de plaquettes sanguines,
(iii) d'introduction d'EPO dans ledit échantillon,
(iv) d'addition de sang ou plasma sanguin lyophilisé supplémentaire,
(v) de polymérisation de l'échantillon à partir de (iv) et
(vi) de culture des cellules dans un milieu convenable qui initie et/ou favorise la croissance et la différentiation des cellules pour donner le tissu désiré.

11. Méthode selon la revendication 10, **caractérisée en ce qu'**on ajoute un(e) ou plusieurs substances, facteurs ou cellules choisis dans le groupe constitué par le GM-CSF, le G-CSF, la GH, les cellules du tissu conjonctif, les fibroblastes, les macrophages et les débris cellulaires.
